# EUROPÄISCHE PATENTSCHRIFT

(11) **EP 0 571 407 B1**
(45) Veröffentlichungstag und Bekanntmachung des Hinweises auf die Patenterteilung: **28.08.1996**
(21) Anmeldenummer: 92903334.8
(22) Anmeldetag: 22.01.1992
(51) Int. Cl.: C12N 15/12, C12N 15/10, C07K 14/00, A61K 38/00, A61K 39/395, G01N 33/53

(54) **KLONIERUNG EINES NEUEN MITGLIEDES DER FAMILIE DER FIBROBLASTEN-WACHSTUMSFAKTOREN (FGF)-REZEPTOREN**
CLONING OF A NEW MEMBER OF THE FIBROBLAST GROWTH FACTOR (FGF) RECEPTOR FAMILY
CLONAGE D'UN NOVEAU MEMBRE DE LA FAMILLE DES RECEPTEURS DES FACTEURS DE CROISSANCE DE FIBROBLASTES (FGF)

(30) Priorität: 12.02.1991 DE 4104240
(43) Veröffentlichungstag der Anmeldung: 01.12.1993
(73) Patentinhaber: BAYER AG, 51368 Leverkusen (DE)
(72) Erfinder: HOLTRICH, Uwe, D-6233 Kelkheim (DE); BRÄUNINGER, Andreas, D-6200 Wiesbaden (DE); STREBHARDT, Klaus, D-6000 Frankfurt a.M. (DE); RÜBSAMEN-WAIGMANN, Helga, D-6232 Bad Soden (DE)
(86) Internationale Anmeldenummer: EP9200127
(87) Internationale Veröffentlichungsnummer: WO9213948

(56) Entgegenhaltungen:
- The EMBO Journal, Band 10, Nr. 6, Juni 1991, J. PARTANEN et al.: "FGFR-4, a novel acidic fibroblast growth factor receptor with a distinct expression pattern", Seiten 1347-1354.
- Proceedings of the National Academy of Science of the USA, Band 87, Nr. 22, November 1990, J. PARTANEN et al.: "Putative tyrosine kinases expressed in K- 562 human leukemia cells", Seiten 8913-8917.
- Proceedings of the National Academy of Science of the USA, Band 86, März 1989, A.F. WILKS: "Two putative protein-tyrosine kinases identified by application of the polymerase chain reaction", Seiten 1603-1607, siehe das ganze Dokument, besonders "Strategy", Figur 1

## Beschreibung

Die vorliegende Erfindung betrifft eine neue Protein-Tyrosin-Kinase, das dafür kodierende Gen sowie ein Verfahren zum Nachweis neuer Protein-Tyrosin-Kinasegene mittels einer PCR-Reaktion.

Die Annahme genetischer Schäden als Ursache der Entstehung von Tumoren wurde durch die Entdeckung von zellulären Genen, "Proto-Onkogene" genannt, bestätigt, die in genetisch modifizierter Weise als sogenannte "Onkogene" das neoplastische Wachstum bedingen. Trotz genetischer Vielfalt der Proto-Onkogene können ihre biochemischen Wirkungsmechanismen in 4 Kategorien zusammengefaßt werden:
1. Phosphorylierung von Proteinen (an den Aminosäuren Threonin, Serin und Tyrosin)
2. Metabolische Regulation
3. Kontrolle der Genexpression
4. Beeinflussung der DNA-Replikation.

Ungefähr ein Drittel aller bisher bekannten Onkogene kodiert für Kinasen, die in Proteinen die Aminosäure Tyrosin phosphorylieren und die daher als Protein-Tyrosin-Kinasen (PTKs) bezeichnet werden. Viele Onkogene, die für Tyrosin-Kinasen kodieren, wurden zuerst im Genom von onkogenen Retroviren entdeckt. So stellt beispielsweise das im Rous-Sarkoma-Virus vorkommende transformierende v-src-Gen eine Modifikation des zellulären Proto-Onkogens c-src dar. Gene, die zu den PTK-Genen homolog sind, wurden daneben auch bei Insekten (z.B. Drosophila melanogaster) und in Hefe (Saccharomyces cerevisiae) gefunden. Der urzeitliche evolutionäre Ursprung und der hohe Grad an Konservierung der PTK-Gene über lange phylogenetische Zeiträume deutet an, daß die Phosphorylierung von Tyrosin eine wichtige Rolle in der zellulären Physiologie von Eukaryonten spielt.

PTK-Gene normaler (nicht transformierter) Zellen können in zwei Kategorien unterteilt werden. Die erste Gruppe kodiert für Oberflächenrezeptoren, die Signale für die Proliferation der Zelle vermitteln. Die Liganden für die folgenden Rezeptoren sind bekannt:
- Epidermal Growth Factor (EGF)-Rezeptor (Cohen, S. et al., 1980, J.Biol.Chem. 225, 4834)
- Insulin-Rezeptor (Ganguli, S. et al., 1985, Curr.Top.Cell.Regulat. 27, 38)
- Colony Stimulating Factor 1(CSF-1)-Rezeptor (Ullrich, A. et al., 1984, Nature 309, 418; Sherr, C.J. et al., 1985, Cell 41, 665)
- Fibroblast Growth Factor (FGF)-Rezeptor (Lee, P.L., 1989, Science 245, 57)

Für zwei weitere Mitglieder, die strukturell zu dieser Familie gehören, sind die Liganden unbekannt: neu (Slamon, DJ. et al., 1987, Science 235, 117) und trk (Bargman, C.J. et al., 1986, Nature 319, 226).

Die zweite Gruppe von PTKs kodiert für Proteine, die zwar mit der Zellmembran assoziiert sind, jedoch keine transmembrane Domäne besitzen: z.B. abl, fgr, src und lck (Hunter, T. and Cooper, J.A., 1985, Ann.Rev.Biochem. 54, 897).

Die PTK-Onkogene wurden ursprünglich entweder als Komponenten von akut transformierenden Retroviren oder durch Transformation von Fibroblasten via Transfektion von DNA aus Tumorzellen identifiziert. Die Produkte folgender retroviraler Onkogene haben PTK-Aktivität: v-src, v-yes, v-fgr, v-fps, v-abl, v-ros, v-fms und v-fes.

Die Gene v-src, v-yes, v-fps, v-ros und v-fms sind onkogene Bestandteile von Hühner-Sarkoma-Viren, v-fgr und v-fes von Katzen-Sarkoma-Viren und v-abl ist ein Onkogen eines Maus-Lymphom-Virus. Eine Analyse der verschiedenen Protein-Tyrosin-Kinasen zeigt eine enge Verwandtschaft über einen Bereich von 260 Aminosäuren, der die katalytische Region umfaßt. In Bereichen, die außerhalb der katalytischen Region liegen, sind die PTKs beträchtlich divergent. Die einzelnen Bereiche entsprechen Domänen, welche die Spezifität der PTKs für ihre Zielproteine bestimmen.

Die Produkte von v-src, v-yes, v-fgr, v-fps, v-fes, v-abl und v-ros zeigen ihre PTK-Aktivität, indem sie in Immunopräzipitat-Reaktionen die schwere Kette des assoziierten Antikörpers und/oder sogar sich selbst phosphorylieren (Autophosphorylierung). In transformierten Zellen liegen diese viralen Proteine nur in geringen Mengen vor, was deren Isolierung und die anschließende Analyse der PTK-Aktivität sehr erschwert.

Im folgenden soll die Bedeutung von Protein-Tyrosin-Kinasen für menschliche Erkrankungen an drei bekannten Protein-Tyrosin-Kinasegenen ausgeführt werden. Das humane HCK-Gen wird in hämatopoietischen Zellen exprimiert. Weiterhin wurde das HCK-Gen der Bande q11-12 des menschlichen Chromosoms 20 zugeordnet. Anomalien in diesem Bereich, dem langen Arm von Chromosom 20, wurden bei zahlreichen Patienten mit hämatologischen Krankheiten beobachtet. So wurden Deletionen, die das HCK-Gen umfassen, bei Patienten mit akuter myeloischer Leukämie, myelodysplastischen Syndromen oder myeloproliferativen Störungen häufig diagnostiziert (Quintrell et al. (1987), Mol. Cell.Biol. 7, 2267). Der chromosomale Lokus von HCK macht es daher wahrscheinlich, daß dieses Gen an der Pathogenese von einigen humanen hämatologischen Krankheiten beteiligt ist.

Das zelluläre Gen, das für den Epidermal Growth Factor (EGF)-Rezeptor kodiert, ist mit dem Onkogen des Avian Erythroblastose-Virus (v-erb B) eng verwandt. In Mammakarzinomen beim Menschen wurde eine DNA-Sequenz identifiziert, die zwar homolog zu v-erb B ist, aber im Vergleich zum EGF-Rezeptor-Gen in amplifizierter Form vorliegt. Die molekulare Analyse zeigte, daß es sich um eine Sequenz handelt, die sehr eng mit dem EGF-Rezeptor verwandt ist. Dieses Onkogen wird ERB B2 bzw. HER2 (human epidermal growth factor receptor 2) genannt. Bei dem Genprodukt handelt es sich um ein neues Mitglied der Gruppe der Protein-Tyrosin-Kinasen. Epidemiologische Untersuchungen haben gezeigt, daß die Amplifizierung von HER2 oft mit aggressiven Mammakarzinomen des Menschen assoziiert ist (Slamon et al. (1987), Science 235, 177). In dieser Studie wurde festgestellt, daß für die Beurteilung der Überlebensrate und die Wahrscheinlichkeit des Rückfalls dieser Patienten mit Brustkrebs die Amplifikationsrate des Oberflächenrezeptors von HER2 ein wesentlicher Prognoseparameter ist. Diese Ergebnisse zeigen, daß das HER2-Gen bei der Pathogenese von Brustkrebs bedeutsam ist.

Die Fibroblast Growth Factor (FGF)-Familie besteht aus mehreren Wachstumsfaktoren, die untereinander homolog sind, Heparin-Bindungseigenschaften besitzen, die Angiogenese fördern und die ein mitogenes Potential gegenüber Zellen epithelialen, mesenchymalen und neuronalen Ursprungs besitzen. Die aberrante Expression von FGFs führt durch einen autokrinen Mechanismus zur Zelltransformation. Darüber hinaus können FGFs das Tumorwachstum und dessen invasives Verhalten beschleunigen, indem der Tumor verstärkt mit Blutgefäßen versorgt wird. FGFs sind auch in der Lage, die Produktion von Proteasen, wie z.B. des Plasminogen-Aktivators zu induzieren.

Beispiele für Mitglieder der FGF-Familie sind das int-2-Genprodukt, das hst-Genprodukt (Kaposi sarcoma-FGF) und der Keratinozyten-Wachstumsfaktor. Die Wirkung der verschiedenen Wachstumsfaktoren wird durch Bindung an einen Oberflächenrezeptor vermittelt, wodurch sich die intrinsische Kinase-Aktivität des Rezeptors erhöht.

Von der immensen klinischen Bedeutung von FGFs und deren Rezeptoren seien hier drei Beispiele herausgegriffen:

Beim Herpes simplex Virus Typ I konnte gezeigt werden, daß der FGF-Rezeptor an der viralen Bindung und Aufnahme in Vertebratenzellen beteiligt ist (Kaner, R.J. et al. (1990), Science 248, 1410).

Auch bei der Entstehung von rheumatischer Arthritis spielt der FGF-Rezeptor eine Rolle. So wurde nachgewiesen, daß Synovialzellen das bFGF-Gen und das bFGF-Rezeptorgen exprimieren und dadurch auf autokrine Weise zur rheumatischen Arthritis stimuliert werden (Melnyk, V. et al. (1990), Arthritis and Rheumatism 33, 493). Weiterhin wurde ein ähnlicher Mechanismus der autokrinen Stimulation über den FGF-Rezeptor auch bei humanen Glioma-Zellinien entdeckt (Morrison, R.S. et al. (1990), Cancer Research 50, 2524).

Partanen et al. in EMBO J. 1991, 10 (6) pp. 1347 - 1354 wird FGFR4 Rezeptor aus Erythroleukämiezellen beschrieben, der sich in mehreren Positionen von dem hier beanspruchten Rezeptor unterscheidet.
Aufgrund der enormen klinischen Bedeutung von Protein-Tyrosin-Kinasen, insbesondere von Protein-Tyrosin-Kinasen aus der FGF-Rezeptorfamilie, bestand somit ein Bedürfnis, neue Protein-Tyrosin-Kinasen sowie Verfahren zu ihrem Nachweis bereitzustellen.

Ein Gegenstand der vorliegenden Erfindung ist somit ein neues TKF (mit dem FGF-Rezeptor verwandte Tyrosin-Kinase) -Rezeptor-Protein, welches dadurch gekennzeichnet ist, daß es a) die in SEQ ID NO: 1 dargestellte Aminosäuresequenz umfaßt Ein Gegenstand der Erfindung ist auch die Verwendung des neuen Proteins, das Teile der in SEQ ID NO: 1 dargestellten Aminosäuresequenz umfaßt oder eines Antikörpers dagegen zur Herstellung eines Mittels für die Tumordiagnostik oder Tumortherapie. Die Verwendung betrifft vorzugweise ein TKF-Rezeptor-Protein, welches die in SEQ ID NO: 1 dargestellte Aminosäuresequenz enthält.

Die in SEQ ID NO: 1 dargestellte Aminosäuresequenz stellt das gesamte TKF-Rezeptor-Protein dar. Dieses Protein weist 802 Aminosäuren auf. Das TKF-Rezeptorgen-Gen können routinemäßig in einem Vektor kloniert werden, so daß dieser Vektor in einer geeigneten Wirtszelle zur Expression gebracht wird, wobei das erfindungsgemäße Protein entsteht. Bevorzugte Wirtszellen sind Mikroorganismen wie E.coli oder Hefe, aber auch höhere Zellen (z.B. Säuger- oder Insektenzellen). Bevorzugte Expressionsvektoren sind z.B. Plasmide, Bakteriophage Lambda für Prokaryonten, Hefevektoren oder virale Vektoren für höhere Zellen (z.B. SV40, Vaccinia, Baculoviren). Bezüglich der Expression des TKF-Gens soll insbesondere auf die bei Sambrook et al. (A Laboratory Manual (1989) Cold Spring Harbor Laboratory Press) genannten Methoden verwiesen werden.

Ein Vergleich der TKF-Rezeptor-Sequenz mit der EMBL-Datenbank ergibt ein hohes Maß an Homologie mit der Familie der Protein-Tyrosin-Kinasen, wobei die engste Verwandtschaft zum humanen Fibroblasten-Wachstumsfaktor-Rezeptor (FGF-Rezeptor) besteht. Das erfindungsgemäße Protein wird daher als TKF (Tyrosine-Kinase related to FGF-Receptor)-Rezeptor bezeichnet.

Es treten folgende Protein-Tyrosin-Kinase spezifische Sequenzcharakteristika auf:
1. Das ATP-Bindungsmotiv:
GlyGluGlyCysPheGly ......(24 Aminosäuren) .... Lys (Aminosäuren 474 - 503)
2. Drei in der katalytischen Domäne von Protein-Tyrosin-Kinasen liegende Aminosäure-Motive, die bei allen Protein-Tyrosin-Kinasen der Rezeptorklasse hinsichtlich der Aminosäuresequenz und des Abstandes der Motive zueinander hoch konserviert sind:

| | |
|---|---|
| HisArgAspLeuAlaAla | (AS 610 - 615) |
| AspPheGly | (AS 630 - 632) |
| ProValLysTrpMetAla | (As 652 - 657) |

Neben der Homologie über die gesamte Sequenz weisen zwei weitere Merkmale der TKF-Rezeptor-Sequenz auf die enge Verwandtschaft zu den FGF-Rezeptoren hin:
1. Die zwischen den Glycinen der ATP-Bindungsregion liegenden Aminosäuren sind bei allen bislang beschriebenen FGF-Rezeptoren gleich und verschieden von denen anderer Protein-Tyrosin-Kinasen.
2. Eine Untergruppe von Rezeptor Protein-Tyrosin-Kinasen unterscheidet sich von anderen Protein-Tyrosin-Kinasen durch ein Aminosäureinsert in der Kinasedomäne. Innerhalb dieser Untergruppe variieren die Längen der Inserts stark. Bei allen bekannten FGF-Rezeptoren ist die Insertlänge jedoch konstant und entspricht mit 19 Aminosäuren der des TKF-Rezeptors.

Weiterhin ein Gegenstand der vorliegenden Erfindung ist eine Nukleinsäure, die für ein erfindungsgemäßes TKF-Rezeptor-Protein oder Teile davon kodiert. Diese Nukleinsäure kann z.B. genomische DNA oder RNA sein. Vorzugsweise handelt es sich dabei jedoch um ein rekombinantes DNA-Molekül.

Weiterhin ein Gegenstand der Erfindung ist eine erfindungsgemäße Nukleinsäure, welche
(a) die in SEQ ID NO: 1 dargestellte kodierende Sequenz,
(b) eine der Sequenz aus (a) im Rahmen der Degeneration des genetischen Codes entsprechende Nukleinsäuresequenz oder
(c) eine mit den Sequenzen aus (a) oder/und (b) unter stringenten Hybridisierungsbedingungen hybridisierende Sequenz enthält.

Unter stringenten Bybridisierungsbedingungen im Sinne der vorliegenden Erfindung versteht man, daß eine Hybridisierung auch nach Waschen bei 55°C in einem wäßrigen Niedrigsalz-Puffer (z.B. 0,2 x SSC) noch auftritt (siehe auch Sambrook, J. et al. (1989), Molecular Cloning. A Laboratory Manual).

Ein weiterer Gegenstand der Erfindung ist ein Vektor, der mindestens eine Kopie einer erfindungsgemäßen Nukleinsäure oder einen Teil davon enthält. Der Vektor kann in Eukaryonten, Prokaryonten oder in Eukaryonten und Prokaryonten replizierbar sein. Er kann ein in das Genom der Wirtszelle integrierbarer Vektor (z.B. Bacteriophage Lambda) oder ein Vektor sein, der extrachromosomal vorliegt (z.B. ein Plasmid). Der erfindungsgemäße Vektor kann durch Subklonierung des TKF-Gens in einen Basisvektor erhalten werden. Derartige Basisvektoren, insbesondere Vektoren mit den zur Proteinexpression erforderlichen Elementen sind einem Fachmann geläufig.

Ein weiterer Gegenstand der vorliegenden Erfindung ist eine Zelle, die mit einer erfindungsgemäßen Nukleinsäure oder einem erfindungsgemäßen Vektor transformiert ist. Die Zelle kann sowohl eine eukaryontische als auch eine prokaryontische Zelle sein. Verfahren zur Transformation von Zellen sind allgemeiner Stand der Technik und brauchen daher nicht erläutert zu werden.

Ein weiterer Gegenstand der vorliegenden Erfindung ist ein Verfahren für den Nachweis neuer Protein-Tyrosin-Kinasegene mittels einer PCR-Reaktion.

Die Polymerase-Kettenreaktion (PCR) eignet sich besonders für Untersuchungen von Wachstumsfaktor-Rezeptor-Genen und Onkogenen, da deren Expression mit gängigen Techniken (Northern Blot) in verschiedenen Fällen unter der Nachweisgrenze liegt. Dies ist bei Protein-Tyrosin-Kinasen häufig der Fall, so daß die PCR speziell für die Suche nach neuen Mitgliedern dieser Gruppe optimiert wurde.

Diese Technik basiert auf der enzymatischen Amplifikation von Sequenzen, die von 2 Oligonukleotidprimern, als 5'- und 3'-Begrenzung fungierend, begrenzt werden. Nach 25 PCR-Zyklen erreicht man einen Amplifikationsgrad von 10⁵-10⁶. Dies verdeutlicht, daß auch äußerst selten vorkommende Transkripte so stark vermehrt werden können, daß man sie anschließend erfolgreich einer Blot Analyse oder der Klonierung zuführen kann.

In einer Analyse der phylogenetischen Verwandtschaft von Protein-Tyrosin-Kinasen konnten die konservierten Bereiche der katalytischen Domänen dieser Enzyme ermittelt werden (Hanks, S. et al., 1988, Science, 241, 42). Aufgrund dieser computeranalytischen Vergleiche können die PTKs im wesentlichen in zwei Kategorien eingeteilt werden: die Rezeptor-Protein-Tyrosin-Kinasen und Protein-Tyrosin-Kinasen ohne extrazelluläre Domäne.

Auf dieser Basis wurden dann entsprechende Primer eingesetzt, um bestimmte Gruppen von Protein-Tyrosin-Kinasen zu untersuchen. Durch Verwendung dieser Primer aus konservierten Regionen wird gewährleistet, daß im Rahmen des PCR-Experiments PTKs der Wachstumsfaktor-Rezeptor-Familie amplifiziert werden können.

Ein Nachteil des bekannten Verfahrens ist jedoch, daß zwei unterschiedliche Primer eingesetzt werden müssen, die zu bekannten Protein-Tyrosin-Kinasegenen homolog sind. Dadurch können Gene, die nur eine Homologie zu einem Primer besitzen, durch eine solche PCR-Reaktion nicht amplifiziert werden.

Ein Gegenstand der Erfindung ist ein Verfahren für den Nachweis neuer Protein-Tyrosin-Kinasegene mittels einer PCR-Reaktion, durch welches die Nachteile des Standes der Technik beseitigt werden. Das erfindungsgemäße Verfahren ist dadurch gekennzeichnet, daß man
(a) aus dem Ausgangsmaterial mRNA nach an sich bekannten Methoden gewinnt,
(b) die mRNA aus Schritt (a) mittels reverser Transkription in cDNA umschreibt,
(c) die cDNA aus Schritt (b) durch terminale Transferase an ihrem 3'-Ende mit einem poly dG-Schwanz versieht,
(d) das aus Schritt (c) resultierende Produkt mittels einer PCR-Reaktion in Anwesenheit von 2 Primern amplifiziert, wobei der erste Primer Sequenzen enthält, die zu hochkonservierten Regionen bekannter Protein-Tyrosin-Kinasegene homolog sind, und der zweite Primer ein oligo dC-Nukleotid ist, und
(e) die resultierenden PCR-Produkte analysiert und charakterisiert.

Dieses Verfahren unterscheidet sich von den bekannten Verfahren dadurch, daß man die zu amplifizierende cDNA durch terminale Transferase an ihrem 3'-Ende mit einem Poly dG-Schwanz versieht, so daß bei der PCR-Reaktion als ein Primer ein Oligo dC-Nukleotid verwendet werden kann, das vorzugsweise eine Länge von 19 bis 25 Nukleotiden besitzt. Somit wird bei dem erfindungsgemäßen Verfahren nur ein Primer (der erste Primer) verwendet, welcher zu hochkonservierten Regionen bekannter Protein-Tyrosin-Kinasegene homologe Sequenzen enthält. Auf diese Weise gelingt es, im Gegensatz zu den Verfahren des Standes der Technik, mittels der PCR-Reaktion Gene zu amplifizieren, bei denen nur ein homologer Primer zur Verfügung steht.

Vorzugsweise verwendet man bei diesem erfindungsgemäßen Verfahren einen ersten Primer, der zu Sequenzen von Protein-Tyrosin-Kinasegenen aus der Familie der Wachstumsfaktorrezeptoren homolog ist. Aufgrund der geringen Konzentration der Protein-Tyrosin-Kinasegen-mRNA im Ausgangsmaterial wird die PCR vorzugsweise in mindestens 20 Zyklen, besonders bevorzugt in etwa 25 Zyklen durchgeführt. Der bei diesem Verfahren verwendete erste Primer (der aus einer hochkonservierten Region bekannter Protein-Tyrosin-Kinasegene stammt) ist vorzugsweise 19 bis 25 Nukleotide lang und enthält besonders bevorzugt einen Bereich aus der in SEQ ID NO: 1 dargestellten Nukleinsäuresequenz oder einer dieser Sequenz im Rahmen der Degeneration des genetischen Codes entsprechenden Nukleotidsequenz, wobei dieser Bereich aus den folgenden Sequenzabschnitten ausgewählt ist:
(a) Nukleotid 1453 - 1470,
(b) Nukleotid 1861 - 1878,
(c) Nukleotid 1921 - 1929,
(d) Nukleotid 1987 - 2004, oder
(e) Nukleotid 2041 - 2058.

Ebenfalls ein Gegenstand der vorliegenden Erfindung ist die Verwendung des TKF-Rezeptorproteins oder des dafür codierenden Gens im Rahmen der Tumordiagnostik. So haben Vorversuche gezeigt, daß die Expression des TKF-Rezeptorgens in Tumoren und korrespondierenden Referenzgeweben unterschiedlich ist. Es besteht daher die Möglichkeit, aus der Expressionshöhe des Gens bzw. des Proteins auf die Zellentartung zu schließen.

Weiterhin ist auch eine therapeutische Anwendung mit monoklonalen Antikörpern gegen den Rezeptor möglich. So könnten beispielsweise radioaktive Antikörper oder mit einem Zellgift gekoppelte Antikörper gegen Krebszellen eingesetzt werden, die den TKF-Rezeptor tragen. Die Herstellung monoklonaler Antikörper gegen den TKF-Rezeptor ist anhand der in SEQ ID NO: 1 dargestellten Aminosäuresequenz des TKF-Rezptorproteins ohne Schwierigkeiten nach bekannten Verfahren möglich. Als Immunogen können das gesamte TKF-Rezeptorprotein oder auch nur kurze (≥ 6 Aminosäuren) Peptidsequenzen daraus verwendet werden.

Schließlich betrifft die Erfindung ein diagnostisches oder therapeutisches Mittel auf Basis eines TKF-Rezeptorproteins, eines dagegen gerichteten Antikörpers oder einer dafür kodierenden Nukleinsäure. Ein derartiges Mittel kann gegebenenfalls bekannte pharmazeutische Verdünnungs-, Füll-, Träger- und Hilfsmittel enthalten.

Die Erfindung soll weiter durch die Sequenzprotokolle SEQ ID NO:1 bis SEQ ID NO:6 zusammen mit den folgenden Beispielen erläutert werden, ohne daß dabei der Umfang der Erfindung beschränkt werden soll.

Es zeigt:
- SEQ ID NO:1: eine 2966 bp lange Nukleinsäuresequenz, die die für das TKF-Rezeptorgen kodierende genetische Information und die 802 AS lange Aminosäuresequenz des TKF-Rezeptorproteins enthält,
- SEQ ID NO:2: die Nukleinsäuresequenz des Oligonukleotidprimers P6(2)
- SEQ ID NO:3: die Nukleinsäuresequenz des Oligonukleotidprimers P5(1),
- SEQ ID NO:4: die Nukleinsäuresequenz des Oligonukleotidprimers P12
- SEQ ID NO:5: die Nukleinsäuresequenz des Oligonukleotidprimers TKF1 und
- SEQ ID NO:6: die Nukleinsäuresequenz des Oligonukleotidprimers T1.

### Beispiel 1

### Isolierung einer Teilsequenz des TKF-Gens

1.1 Es wurde gemäß dem Verfahren von Chirgwin et al. (Bio-chemistry 18 (1979), 5294) RNA aus humanem Lungengewebe isoliert. Mit dieser RNA wurde eine PTK-spezifische cDNA-Synthese durchgeführt. Die Reaktionsbedingungen waren wie folgt:
   50 mmol/l Tris/HCl, pH 8,3 (22°C)
   75 mmol/l KCl
   10 mmol/l Dithiotreitol (DTT)
   3 mmol/l MgCl₂
   500 µmol/l dNTP-Lösung
   0,2 µmol/l Oligonukleotid-Primer P6(2) mit der in SEQ ID NO:2 dargestellten Nukleinsäuresequenz (diese Sequenz entspricht den Nukleotiden 2044 bis 2063 der in SEQ ID NO:1 dargestellten Sequenz).
   3,0 µg Gesamt-RNA
   100 µg/ml Rinderserumalbumin
   10 Einheiten Reverse Transkriptase (aus Moloney - Murine Leukemia virus)
   Das obige Reaktionsgemisch wurde in 20 µl Reaktionsvolumen eine Stunde lang bei 37°C inkubiert.
1.2 Die Amplifizierung der cDNA aus 1.1 erfolgte mit zwei zu hoch konservierten Regionen von PTK-Genen komplementären Primern (P6(2) und P5(1)). Die Nukleinsäuresequenz des Primers P6(2) ist bereits oben angegeben. Die Sequenz von P5(1) ist in SEQ ID NO:3 dargestellt (diese Sequenz entspricht den Nukleotiden 1855 bis 1874 der in SEQ ID NO:1 dargestellten Sequenz).
   Reaktionsbedingungen:
   8,3 mmol/l Tris/HCl, pH 8,8 (22°C)
   41,7 mmol/l KCl
   1,25 mmol/l MgCl₂
   0,01 % Gelatine
   166,7 µmol/l dNTP-Lösung
   0,2 µmol/l Primer P6(2) und P5(1)
   5 Einheiten Taq-Polymerase
   10 µl cDNA-Syntheseansatz (aus der PTK-spezifischen cDNA-Synthese)

   Das Reaktionsvolumen war 60 µl. Die Bedingungen für einen PCR-Zyklus waren wie folgt:
   96°C Denaturierung für 1 Minute,
   40°C Hybridisierung für 2 Minuten,
   70°C Elongation für 3 Minuten.
   Es wurden insgesamt 25 Zyklen durchgeführt.

Auf diese Weise wurde ein 208 bp langes DNA-Fragment erhalten, das nach üblichen Methoden kloniert und sequenziert wurde (Sambrook, J. et al., Molecular Cloning, A Laboratory Manual, Cold Spring Harbor Laboratory 1989). Die Nukleinsäuresequenz dieses DNA-Fragments entspricht den Nukleotiden 1855 bis 2063 der in SEQ ID NO:1 dargestellten Sequenz.

### Beispiel 2

Isolierung einer längeren Teil sequenz des TKF-Rezeptorgens mit Hilfe der Anker-PCR
2.1 Es wurde ein homopolymerisches G-Tailing von cDNA zur 5'-terminalen Verlängerung des in Beispiel 1 erhaltenen PCR-Produkts durchgeführt.
   Reaktionsbedingungen:
   1 µl cDNA (0,5 bis 100 pg aus Beispiel 1)
   100 mmol/l Kaliumkakodylat pH 7,1 (22°C)
   1 mmol/l CoCl₂
   0,1 mmol/l DTT
   50 µg/ml Rinderserumalbumin
   50 Einheiten terminale Transferase
   25 mmol/l Tris/HCl, pH 7,1 (22°C)
   100 µmol/l dGTP

   Die Durchführung erfolgte in einem Reaktionsvolumen von 20 µl. Der Ansatz wurde bis zum Reaktionsbeginn auf Eis gehalten. Die cDNA für die Tailing-Reation wurde wie folgt hergestellt:
   Zunächst erfolgte eine Entfernung der restlichen, nicht eingebauten dNTPs aus dem cDNA-Syntheseansatz durch Ultrafiltration mit Millipore UFC3 LGC 25-Filtern. Vor der Reaktion wurde die cDNA 3 Minuten lang in kochendem Wasser denaturiert, dann auf Eis abgekühlt und zum Reaktionsansatz pipettiert. Das Reaktionsgemisch wurde 20 Minuten bei 37°C inkubiert. Durch Erhitzen für 5 Minuten auf 65°C wurde die Reaktion abgestoppt.
2.2 Als nächster Schritt wurde eine PCR-Reaktion mit Primern durchgeführt, wovon der eine mit einer zum poly dG-Schwanz komplementären Oligo-dC-Sequenz versehen war. Eine derartige PCR wird als Anker-PCR-bezeichnet. Der zweite Primer (P6(2)) war zu konservierten Regionen in Protein-Tyrosin-Kinasen komplementär.
   Als Oligo-dC-Primer wude das Oligonukleotid P12 verwendet, dessen Nukleinsäuresequenz in SEQ ID NO:4 dargestellt ist.
   Die Polymerasekettenreaktion wurde in zwei Amplifikationsrunden durchgeführt. Die Reaktionsbedingungen der ersten Amplifikationsrunden waren wie folgt:
   10 mmol/l Tris/HCl, pH 8,8 (22°C)
   50 mmol/l KCl
   1,5 mmol/l MgCl₂
   0,01 % Gelatine
   200 µmol/l dNTP-Lösung
   0,2 µmol/l der Primer P6(2) und P12
   5 Einheiten Taq-Polymerase
   10 µl Tailing-Reaktionsprodukt (0,5 bis 100 pg)

   Das Reaktionsvolumen war 50 µl. Die Bedingungen bei einem Zyklus der PCR-Reaktion waren wie folgt:
   Denaturierung bei 96°C für 1 Minute,
   Hybridisierung bei 40°C für 2 Minuten und
   Elongation bei 70°C für 3 Minuten.
   Insgesamt wurden 25 Zyklen durchgeführt.

Für die zweite Amplifikationsrunde wurde 1 µl PCR-Reaktionsprodukt aus der ersten Runde in einen frischen PCR-Ansatz gegeben und unter den gleichen Bedingungen wie oben nochmals in 25 Zyklen amplifiziert. Zur Erhöhung der Spezifität wird ein Primerwechsel von P6(2)/P12 auf TKF1/P12 vorgenommen. Die Nukleinsäuresequenz des Primers TKF1 ist in SEQ ID NO:5 dargestellt (diese Sequenz entspricht den Nukleotiden 1875 bis 1902 der in SEQ ID NO:1 dargestellten Sequenz).

Bei der oben beschriebenen Prozedur wurde bei der Anker-PCR neben der Teil sequenz des TKF-Gens eine neue Protein-Tyrosin-Kinase-Sequenz gefunden, die bisher noch nicht charakterisiert wurde.

### Beispiel 3

### Isolierung einer TKF-Rezeptor-cDNA

Es wurde eine cDNA-Bank aus poly A⁺ RNA von humanem Lungengewebe mit 1,8 x 10⁶ rekombinanten Klonen etabliert. Die Isolierung der mRNA erfolgte gemäß Aviv und Leder (Proc.Natl. Acad.Sci. USA 69 (1972), 1408). Die cDNA-Klonierung erfolgte gemäß Gubler und Hoffman (Gene 25 (1983), 263). Diese Genbank wurde mittels einer DNA-Sonde durchmustert. Die DNA-Sonde wurde durch PCR hergestellt. Als Primer für die PCR wurden die Oligonukleotide P6(2) und T1 verwendet. Die Nukleinsäuresequenz für T1 ist in SEQ ID NO:6 dargestellt (diese Sequenz entspricht den Nukleotiden 1846 bis 1866 der in SEQ ID NO:1 dargestellten Sequenz).

Reaktionsbedingungen:
1 ng TKF-Rezeptor Insert (aus Beispiel 2)
10 mmol/l Tris/HCl, pH 8,8 (22°C)
50 mmol/l KCl
1,5 mmol/l MgCl₂
0,01 % Gelatine
0,8 µmol/l α³² P-dCTP (6000 Ci/mmol)
0,8 µmol/l dATP/dGTP/dTTP
0,2 µmol/l der Primer P6(2) und T1
2,5 Einheiten Taq-Polymerase

Das Reaktionsvolumen war 25 µl. Die Durchführung der Reaktion erfolgte in 20 Zyklen bei den oben (in Beispiel 2) genannten PCR-Bedingungen.

Zum Nachweis eines positiven Klons wurde cDNA aus der Genbank auf Filtern immobilisiert und an die radioaktiv markierte DNA-Sonde (1 x 10⁶ dpm/ml Lösung bei einer spezifischen Sondenaktivität von 5 x 10⁹ dpm/µg DNA) hybridisiert. Die Hybridisierungstemperatur war 42°C.

Hybridisierungslösung:
5 x SSC
0,02 mol/l Tris/HCl, pH 7,6 (22°C)
1 x Denhardt-Lösung
10 % Dextransulfat
0,1 % SDS
100 µg/ml Heringssperma DNA
50 % Formamid

Als Waschlösung wurde 2 x SSC, 0,1 % SDS bei einer Waschtemperatur von 42°C eingesetzt.

Es wurde ein positiver Klon gefunden und charakterisiert. Er enthielt die in SEQ ID NO:1 dargestellte Nukleinsäuresequenz.

30 60
CCA GCT TGG GTC CCT GAG AGC TGT GAG AAG GAG ATG CGG CTG CTG CTG GCC CTG TTG GGG
Met Arg Leu Leu Leu Ala Leu Leu Gly

90 120
GTC CTG CTG AGT GTG CCT GGG CCT CCA GTC TTG TCC CTG GAG GCC TCT GAG GAA GTG GAG
Val Leu Leu Ser Val Pro Gly Pro Pro Val Leu Ser Leu Glu Ala Ser Glu Glu Val Glu

150 180
CTT GAG CCC TGC CTG GCT CCC AGC CTG GAG CAG CAA GAG CAG GAG CTG ACA GTA GCC CTT
Leu Glu Pro Cys Leu Ala Pro Ser Leu Glu Gln Gln Glu Gln Glu Leu Thr Val Ala Leu

210 240
GGG CAG CCT GTG CGT CTG TGC TGT GGG CGG GCT GAG CGT GGT GGC CAC TGG TAC AAG GAG
Gly Gln Pro Val Arg Leu Cys Cys Gly Arg Ala Glu Arg Gly Gly His Trp Tyr Lys Glu

270 300
GGC AGT CGC CTG GCA CCT GCT GGC CGT GTA CGG GGC TGG AGG GGC CGC CTA GAG ATT GCC
Gly Ser Arg Leu Ala Pro Ala Gly Arg Val Arg Gly Trp Arg Gly Arg Leu Glu Ile Ala

330 360
AGC TTC CTA CCT GAG GAT GCT GGC CGC TAC CTC TGC CTG GCA CGA GGC TCC ATG ATC GTC
Ser Phe Leu Pro Glu Asp Ala Gly Arg Tyr Leu Cys Leu Ala Arg Gly Ser Met Ile Val

390 420
CTG CAG AAT CTC ACC TTG ATT ACA GGT GAC TCC TTG ACC TCC AGC AAC GAT GAT GAG GAC
Leu Gln Asn Leu Thr Leu Ile Thr Gly Asp Ser Leu Thr Ser Ser Asn Asp Asp Glu Asp

450 480
CCC AAG TCC CAT AGG GAC CTC TCG AAT AGG CAC AGT TAC CCC CAG CAA GCA CCC TAC TGG
Pro Lys Ser His Arg Asp Leu Ser Asn Arg His Ser Tyr Pro Gln Gln Ala Pro Tyr Trp

510 540
ACA CAC CCC CAG CGC ATG GAG AAG AAA CTG CAT GCA GTA CCT GCG GGG AAC ACC GTC AAG
Thr His Pro Gln Arg Met Glu Lys Lys Leu His Ala Val Pro Ala Gly Asn Thr Val Lys

570 600
TTC CGC TGT CCA GCT GCA GGC AAC CCC ACG CCC ACC ATC CGC TGG CTT AAG GAT GGA CAG
Phe Arg Cys Pro Ala Ala Gly Asn Pro Thr Pro Thr Ile Arg Trp Leu Lys Asp Gly Gln

630 660
GCC TTT CAT GGG GAG AAC CGC ATT GGA GGC ATT CGG CTG CGC CAT CAG CAC TGG AGT CTC
Ala Phe His Gly Glu Asn Arg Ile Gly Gly Ile Arg Leu Arg His Gln His Trp Ser Leu

690 720
GTG ATG GAG AGC GTG GTG CCC TCG GAC CGC GGC ACA TAC ACC TGC CTG GTA GAG AAC GCT
Val Met Glu Ser Val Val Pro Ser Asp Arg Gly Thr Tyr Thr Cys Leu Val Glu Asn Ala

750 780
GTG GGC AGC ATC CGT TAT AAC TAC CTG CTA GAT GTG CTG GAA CGG TCA CCG CAC CGG CCC
Val Gly Ser Ile Arg Tyr Asn Tyr Leu Leu Asp Val Leu Glu Arg Ser Pro His Arg Pro

810 840
ATC CTG CAG GCC GGG CTC CCG GCC AAC ACC ACA GCC GTG GTG GGC AGC GAC GTG GAG CTG
Ile Leu Gln Ala Gly Leu Pro Ala Asn Thr Thr Ala Val Val Gly Ser Asp Val Glu Leu

870 900
CTG TGC AAG GTG TAC AGC GAT GCC CAG CCC CGC ATC CAG TGG CTG AAG CAC ATC GCC ATC
Leu Cys Lys Val Tyr Ser Asp Ala Gln Pro Arg Ile Gln Trp Leu Lys His Ile Ala Ile

930 960
AAC GGC AGC AGC TTC GGA GCC GAC GGT TTC CCC TAT GTG CAA GTC CTA GAG ACT GCA GAC
Asn Gly Ser Ser Phe Gly Ala Asp Gly Phe Pro Tyr Val Gln Val Leu Glu Thr Ala Asp

990 1020
ATC AAT AGC TCA GAG GTG GAG GTC CTG TAC CTG CGG AAC GTG TCA GCC GAG GAC GCA GGC
Ile Asn Ser Ser Glu Val Glu Val Leu Tyr Leu Arg Asn Val Ser Ala Glu Asp Ala Gly

1050 1080
GAG TAC ACC TGC CTC GCA GGC AAT TCC ATC GGC CTC TCC TAC CAG TCT GCC TGG CTC ACG
Glu Tyr Thr Cys Leu Ala Gly Asn Ser Ile Gly Leu Ser Tyr Gln Ser Ala Trp Leu Thr

1110 1140
GTG CTG CCA GAG GAG GAC CCC ACA TGG ACC GCA GCA GCG CCC GAG GCC AGG TAT ACG GAC
Val Leu Pro Glu Glu Asp Pro Thr Trp Thr Ala Ala Ala Pro Glu Ala Arg Tyr Thr Asp

1170 1200
ATC ATC CTG TAC GCG TCG GGC TCC CTG GCC TTG GCT GCG CTC CTG CTG CTG GCC GGG CTG
Ile Ile Leu Tyr Ala Ser Gly Ser Leu Ala Leu Ala Ala Leu Leu Leu Leu Ala Gly Leu

1230 1260
TAT CGA GGG CAG GCG CTC CAC GGC CGG CAC CCC CGC CCG CCC GCC ACT GTG CAG AAG CTC
Tyr Arg Gly Gln Ala Leu His Gly Arg His Pro Arg Pro Pro Ala Thr Val Gln Lys Leu

1290 1320
TCC CGC TTC CCT CTG GCC CGA CAG TTC TCC CTG GAG TCA GGC TCT TCC GGC AAG TCA AGC
Ser Arg Phe Pro Leu Ala Arg Gln Phe Ser Leu Glu Ser Gly Ser Ser Gly Lys Ser Ser

1350 1380
TCA TCC CTG GTA CGA GGC GTG CGT CTC TCC TCC AGC GGC CCC GCC TTG CTC GCC GGC CTC
Ser Ser Leu Val Arg Gly Val Arg Leu Ser Ser Ser Gly Pro Ala Leu Leu Ala Gly Leu

1410 1440
GTG AGT CTA GAT CTA CCT CTC GAC CCA CTA TGG GAG TTC CCC CGG GAC AGG CTG GTG CTT
Val Ser Leu Asp Leu Pro Leu Asp Pro Leu Trp Glu Phe Pro Arg Asp Arg Leu Val Leu

1470 1500
GGG AAG CCC CTA GGC GAG GGC TGC TTT GGC CAG GTA GTA CGT GCA GAG GCC TTT GGC ATG
Gly Lys Pro Leu Gly Glu Gly Cys Phe Gly Gln Val Val Arg Ala Glu Ala Phe Gly Met

1530 1560
GAC CCT GCC CGG CCT GAC CAA GCC AGC ACT GTG GCC GTC AAG ATG CTC AAA GAC AAC GCC
Asp Pro Ala Arg Pro Asp Gln Ala Ser Thr Val Ala Val Lys Met Leu Lys Asp Asn Ala

1590 1620
TCT GAC AAG GAC CTG GCC GAC CTG GTC TCG GAG ATG GAG GTG ATG AAG CTG ATC GGC CGA
Ser Asp Lys Asp Leu Ala Asp Leu Val Ser Glu Met Glu Val Met Lys Leu Ile Gly Arg

1650 1680
CAC AAG AAC ATC ATC AAC CTG CTT GGT GTC TGC ACC CAG GAA GGG CCC CTG TAC GTG ATC
His Lys Asn Ile Ile Asn Leu Leu Gly Val Cys Thr Gln Glu Gly Pro Leu Tyr Val Ile

1710 1740
GTG GAG TGC GCC GCC AAG GGA AAC CTG CGG GAG TTC CTG CGG GCC CGG CGC CCC CCA GGC
Val Glu Cys Ala Ala Lys Gly Asn Leu Arg Glu Phe Leu Arg Ala Arg Arg Pro Pro Gly

1770 1800
CCC GAC CTC AGC CCC GAC GGT CCT CGG AGC AGT GAG GGG CCG CTC TCC TTC CCA GTC CTG
Pro Asp Leu Ser Pro Asp Gly Pro Arg Ser Ser Glu Gly Pro Leu Ser Phe Pro Val Leu

1830 1860
GTC TCC TGC GCC TAC CAG GTG GCC CGA GGC ATG CAG TAT CTG GAG TCC CGG AAG TGT ATC
Val Ser Cys Ala Tyr Gln Val Ala Arg Gly Met Gln Tyr Leu Glu Ser Arg Lys Cys Ile

1890 1920
CAC CGG GAC CTG GCT GCC CGC AAT GTG CTG GTG ACT GAG GAC AAT GTG ATG AAG ATT GCT
His Arg Asp Leu Ala Ala Arg Asn Val Leu Val Thr Glu Asp Asn Val Met Lys Ile Ala

1950 1980
GAC TTT GGG CTG GCC CGC GGC GTC CAC CAC ATT GAC TAC TAT AAG AAA ACC AGC AAC GGC
Asp Phe Gly Leu Ala Arg Gly Val His His Ile Asp Tyr Tyr Lys Lys Thr Ser Asn Gly

2010 2040
CGC CTG CCT GTG AAG TGG ATG GCG CCC GAG GCC TTG TTT GAC CGG GTG TAC ACA CAC CAG
Arg Leu Pro Val Lys Trp Met Ala Pro Glu Ala Leu Phe Asp Arg Val Tyr Thr His Gln

2070 2100
AGT GAC GTG TGG TCT TTT GGG ATC CTG CTA TGG GAG ATC TTC ACC CTC GGG GGC TCC CCG
Ser Asp Val Trp Ser Phe Gly Ile Leu Leu Trp Glu Ile Phe Thr Leu Gly Gly Ser Pro

2130 2160
TAT CCT GGC ATC CCG GTG GAG GAG CTG TTC TCG CTG CTG CGG GAG GGA CAT CGG ATG GAC
Tyr Pro Gly Ile Pro Val Glu Glu Leu Phe Ser Leu Leu Arg Glu Gly His Arg Met Asp

2190 2220
CGA CCC CCA CAC TGC CCC CCA GAG CTG TAC GGG CTG ATG CGT GAG TGC TGG CAC GCA GCG
Arg Pro Pro His Cys Pro Pro Glu Leu Tyr Gly Leu Met Arg Glu Cys Trp His Ala Ala

2250 2280
CCC TCC CAG AGG CCT ACC TTC AAG CAG CTG GTG GAG GCG CTG GAC AAG GTC CTG CTG GCC
Pro Ser Gln Arg Pro Thr Phe Lys Gln Leu Val Glu Ala Leu Asp Lys Val Leu Leu Ala

2310 2340
GTC TCT GAG GAG TAC CTC GAC CTC CGC CTG ACC TTC GGA CCC TAT TCC CCC TCT GGT GGG
Val Ser Glu Glu Tyr Leu Asp Leu Arg Leu Thr Phe Gly Pro Tyr Ser Pro Ser Gly Gly

2370 2400
GAC GCC AGC AGC ACC TGC TCC TCC AGC GAT TCT GTC TTC AGC CAC GAC CCC CTG CCA TTG
Asp Ala Ser Ser Thr Cys Ser Ser Ser Asp Ser Val Phe Ser His Asp Pro Leu Pro Leu

2430 2460
GGA TCC AGC TCC TTC CCC TTC GGG TCT GGG GTG CAG ACA TGA GCA AGG CTC AAG GCT GTG
Gly Ser Ser Ser Phe Pro Phe Gly Ser Gly Val Gln Thr End

2490 2520
CAG GCA CAT AGG CTG GTG GCC TTG GGC TTG GGG CTC AGC CAC AGC CTG ACA CAG TGC TCG

2550 2580
ACC TTG ATA GCA TGG GGC CCC TGG CAG AGT TGC TGT GCC GTG TCC AAG GCC GTG CCC TTG

2610 2640
CCC TTG GAG CTG CCG TGC CTG TGT CCT GAT GGC CCA AAT GTC AGG GTT CTG CTC GGC TTC

2670 2700
TTG GAC CTT GGC GCT TAG TCC CCA TCC CGG GTT TGG CTG AGC CTG GCT GGA GAG CTG CTA

2730 2760
TGC TAA ACC TCC TGC CTC CCA ATA CCA GCA GGA GGT TCT GGG CCT CTG AAC CCC CTT TCC

2790 2820
CCA CAC CTC CCC CTG CTG CTG CTG CCC CAG CGT CTT GAC GGG AGC ATT GGC CCC TGA GCC

2850 2880
CAG AGA AGC TGG AAG CCT GCC GAA AAC AGG AGC AAA TGG CGT TTT ATA AAT TAT TTT TTT

2910 2940
GAA ATA AAG CTC TGT GTG CCT GGG TCA AAA AAA AAA AAA AAA AAA AAA AAA AAA AAA AAA

AAA AAA AAA AAA AAA AAA AAA AAA AA

### SEQ ID NO:2

- ART DER SEQUENZ:: Nukleinsäuresequenz
- NAME UND HERKUNFT:: Synthetischer Oligonukleotidprimer P6(2)
- LÄNGE:: 20 Nukleotide

ATCCCATAAC ACCACACGTC

### SEQ ID NO:3

- ART DER SEQUENZ:: Nukleinsäuresequenz
- NAME UND HERKUNFT:: Synthetischer Oligonukleotidprimer P5(1)
- LÄNGE:: 20 Nukleotide

TTTGTCCACC GAGACCTGGC

### SEQ ID NO:4

- ART DER SEQUENZ:: Nukleinsäuresequenz
- NAME UND HERKUNFT:: Synthetischer Oligonukleotidprimer P12
- LÄNGE:: 33 Nukleotide

GATTTCAGAG AACTAAACCC CCCCCCCCCC CCC

### SEQ ID NO:5

- ART DER SEQUENZ:: Nukleinsäuresequenz
- NAME UND HERKUNFT:: Synthetischer Oligonukleotidprimer TKF1
- LÄNGE:: 28 Nukleotide

GTCCTCAGTC ACCAGCACAT TGCGGGCA

### SEQ ID NO:6

- ART DER SEQUENZ:: Nukleinsäuresequenz
- NAME UND HERKUNFT:: Synthetischer Oligonukleotidprimer T1
- LÄNGE:: 21 Nukleotide

TCCCGGAAGT GTATCCACCG G

## Patentansprüche (Patentansprüche für folgende(n) Vertragsstaat(en): AT, BE, CH, LI, DE, DK, FR, GB, IT, LU, NL, SE)

1. TKF-Rezeptor Protein,
**dadurch gekennzeichnet**,
daß es die in SEQ ID NO: 1 dargestellte Aminosäuresequenz umfaßt.

2. Nukleinsäure,
**dadurch gekennzeichnet**,
daß sie für ein TKF-Rezeptor Protein nach Anspruch 1 kodiert und
(a) die in SEQ ID NO: 1 dargestellte kodierende Sequenz oder
(b) eine der Sequenz aus (a) im Rahmen der Degeneration des genetischen Codes entsprechende Nukleinsäuresequenz enthält.

3. Nukleinsäure nach Anspruch 2,
**dadurch gekennzeichnet**,
daß sie ein rekombinantes DNA-Molekül ist.

4. Vektor,
**dadurch gekennzeichnet**,
daß er mindestens eine Kopie einer Nukleinsäure nach Anspruch 2 oder 3 oder einen Teil davon enthält.

5. Zelle,
**dadurch gekennzeichnet**,
daß sie mit einer Nukleinsäure nach Anspruch 2 oder 3 oder einem Vektor nach Anspruch 4 transformiert ist.

6. Verwendung eines Proteins, das (a) die in SEQ ID NO: 1 dargestellte Aminosäuresequenz oder (b) Varianten der Sequenz aus (a) mit einer Homologie von mindestens 85 % auf Aminosäureebene umfaßt, oder eines dagegen gerichteten Antikörpers zur Herstellung eines Mittels für die Tumordiagnostik oder Tumortherapie.

7. Verwendung einer Nukleinsäure, die (a) die in SEQ ID NO: 1 dargestellte kodierende Sequenz, (b) eine der Sequenz aus (a) im Rahmen der Degeneration des genetischen Codes entsprechende Nukleinsäuresequenz oder (c) eine mit den Sequenzen aus (a) oder/und (b) unter stringenten Hybridisierungsbedingungen hybridisierende Sequenz enthält, wobei stringente Hybridisierungsbedingungen bedeuten, daß eine Hybridisierung nach Waschen bei 55°C in 0,2 x SSC-Puffer auftritt, zur Herstellung eines Mittels für die Tumordiagnostik oder Tumortherapie.

## Patentansprüche (Patentansprüche für folgende(n) Vertragsstaat(en): ES, GR)

1. Verwendung eines Proteins, das (a) die in SEQ ID No. 1 dargestellte Aminosäuresequenz oder (b) Varianten der Sequenz aus (a) mit einer Homologie von mindestens 85 % auf Aminosäureebene umfaßt, oder eines dagegen gerichteten Antikörpers zur Herstellung eines Mittels für die Tumordiagnostik oder Tumortherapie.

2. Verwendung einer Nukleinsäure, die (a) die in SEQ ID No. 1 dargestellte kodierende Sequenz, (b) eine der Sequenz aus (a) im Rahmen der Degeneration des genetischen Codes entsprechende Nukleinsäuresequenz oder (c) eine mit den Sequenzen aus (a) oder/und (b) unter stringenten Hybridisierungbedingungen hybridisierende Sequenz enthält, wobei stringente Hybridisierungsbedingungen bedeuten, daß eine Hybridisierung nach Waschen bei 55°C in 0,2 x SSC-Puffer auftritt, zur Herstellung eines Mittels für die Tumordiagnostik oder Tumortherapie.

## Claims (Claims for the following Contracting State(s): AT, BE, CH, DE, DK, FR, GB, IT, LI, LU, NL, SE)

1. TKF receptor protein,
characterized in that
it comprises the amino acid sequence depicted in SEQ ID NO: 1.

2. Nucleic acid,
characterized in that
it encodes a TKF receptor protein according to Claim 1 and
(a) contains the coding sequence depicted in SEQ ID NO: 1 or
(b) contains a nucleic acid sequence which corresponds to the sequence from (a) allowing for the degeneracy of the genetic code.

3. Nucleic acid according to Claim 2,
characterized in that
it is a recombinant DNA molecule.

4. Vector,
characterized in that
it contains at least one copy of a nucleic acid according to Claim 2 or 3 or a part thereof.

5. Cell,
characterized in that
it is transformed with a nucleic acid according to Claim 2 or 3 or a vector according to Claim 4.

6. Use of a protein which comprises (a) the amino acid sequence depicted in SEQ ID NO: 1 or (b) variants of the sequence from (a) having an homology of at least 85% at the amino acid level, or of an antibody which is directed against it for preparing a composition for the diagnosis or therapy of tumours.

7. Use of a nucleic acid which contains (a) the coding sequence depicted in SEQ ID NO: 1, (b) a nucleic acid sequence which corresponds to the sequence from (a) allowing for the degeneracy of the genetic code, or (c) a sequence which hybridizes with the sequences from (a) and/or (b) under stringent hybridization conditions, where stringent hybridization conditions denote that hybridization occurs after washing in 0.2 × SSC buffer at 55°C, for preparing a composition for the diagnosis or therapy of tumours.

## Claims (Claims for the following Contracting State(s): ES, GR)

1. Use of a protein which comprises (a) the amino acid sequence depicted in SEQ ID No. 1 or (b) variants of the sequence from (a) having an homology of at least 85% at the amino acid level, or of an antibody which is directed against it for preparing a composition for the diagnosis or therapy of tumours.

2. Use of a nucleic acid which contains (a) the coding sequence depicted in SEQ ID No. 1, (b) a nucleic acid sequence which corresponds to the sequence from (a) allowing for the degeneracy of the genetic code, or (c) a sequence which hybridizes with the sequences from (a) and/or (b) under stringent hybridization conditions, where stringent hybridization conditions denote that hybridization occurs after washing in 0.2 × SSC buffer at 55°C, for preparing a composition for the diagnosis or therapy of tumours.

## Revendications (Revendications pour l'(les) Etat(s) contractant(s) suivant(s): AT, BE, CH, DE, DK, FR, GB, IT, LI, LU, NL, SE)

1. Protéine du récepteur TKF,
caractérisée
en ce qu'elle comprend la séquence d'acides aminés représentée dans SEQ ID NO: 1.

2. Acide nucléique
caractérisé
en ce qu'il code pour une protéine du récepteur TKF selon la revendication 1 et contient
(a) la séquence codante représentée dans SEQ ID NO: 1 ou
(b) une séquence d'acides nucléiques correspondant à la séquence de (a) dans le cadre de la dégénérescence du code génétique.

3. Acide nucléique selon la revendication 2,
caractérisé
en ce qu'il est une molécule d'ADN recombiné.

4. Vecteur
caractérisé
en ce qu'il contient au moins une copie d'un acide nucléique selon la revendication 2 ou 3 ou une partie de ce dernier.

5. Cellule
caractérisée
en ce qu'elle a été transformée avec un acide nucléique selon la revendication 2 ou 3 ou avec un vecteur selon la revendication 4.

6. Utilisation d'une protéine qui comprend (a) la séquence d'acides aminés représentée dans SEQ ID NO: 1 ou (b) des variantes de la séquence de (a) avec une homologie d'au moins 85% sur le plan des acides aminés, ou encore d'un anticorps dirigé contre elle pour la préparation d'un agent pour le diagnostic tumoral ou pour la thérapie tumorale.

7. Utilisation d'un acide nucléique qui contient (a) la séquence codante représentée dans SEQ ID NO: 1, (b) une séquence d'acides nucléiques correspondant à la séquence de (a) dans le cadre de la dégénérescence du code génétique ou (c) une séquence s'hybridant avec les séquences de (a) et/ou de (b) dans des conditions d'hybridation strictes, des conditions d'hybridation strictes signifiant qu'une hybridation apparaît après lavage à 55°C dans un tampon 0,2 x SSC, pour la préparation d'un agent pour le diagnostic tumoral ou pour la thérapie tumorale.

## Revendications (Revendications pour l'(les) Etat(s) contractant(s) suivant(s): ES, GR)

1. Utilisation d'une protéine qui comprend (a) la séquence d'acides aminés représentée dans SEQ ID NO: 1 ou (b) des variantes de la séquence de (a) avec une homologie d'au moins 85% sur le plan des acides aminés, ou encore d'un anticorps dirigé contre elle pour la préparation d'un agent pour le diagnostic tumoral ou pour la thérapie tumorale.

2. Utilisation d'un acide nucléique qui contient (a) la séquence codante représentée dans SEQ ID NO: 1, (b) une séquence d'acides nucléiques correspondant à la séquence de (a) dans le cadre de la dégénérescence du code génétique ou (c) une séquence s'hybridant avec les séquences de (a) et/ou (b) dans des conditions d'hybridation strictes, des conditions d'hybridation strictes signifiant qu'une hybridation apparaît après lavage à 55°C dans un tampon 0,2 x SSC, pour la préparation d'un agent pour le diagnostic tumoral ou pour la thérapie tumorale.
